# EUROPEAN PATENT APPLICATION

(11) **EP 2 198 857 A1**
(43) Date of publication of application: **23.06.2010**
(21) Application number: 08022146.8
(22) Date of filing: 19.12.2008
(51) Int. Cl.: A61K 9/20

(54) **Oral dispersible tablet**

(71) Applicant: Ratiopharm GmbH, 89079 Ulm (DE)
(72) Inventor: Winter, Swen, 89134 Blaustein (DE); Scheiwe, Max-Werner, Dr., 79689 Maulberg (DE)
(74) Representative: Teipel, Stephan

(57) **Abstract**

The present invention relates to an oral dispersible tablet comprising at least one filler selected from sugars and sugar alcohols, and microcrystalline cellulose.

## Description

The present invention relates to an oral dispersible tablet comprising at least one filler selected from sugars and sugar alcohols, and microcrystalline cellulose.

Oral dispersible tablets when placed in the oral cavity rapidly dissolve or disintegrate without water and therefore are a valuable tool to treat patients, which have problems taking normal tablets. The tablets can be taken with or without a small amount of water. They disintegrate fast in the mouth. They are also a good choice for medicines, which have to be taken when people are, for example, travelling and water for swallowing a normal tablet is not available.

The design of an oral dispersible tablet requires a significant amount of research work in order to develop a tablet having enough porosity inside for fast dissolution or fast disintegration while maintaining the mechanical strength required, for example, to pack the tablets into blisters. Current technologies involved in the manufacturing of fast-dissolving tablets utilize complicated processing techniques such as freeze-drying, moulding and sublimation or require specialized excipients, in particular disintegrants and superdisintegrants. For example, WO 2007/113856 discloses a directly compressible composite for an orally disintegrating tablet comprising at least one water-soluble excipient and calcium silicate prepared by co-processing. In example 17 of this document a tablet is prepared by first co-processing mannitol and calcium silicate and then blending the obtained composite with xylitol, microcrystalline cellulose and further ingredients. The ration of mannitol and xylitol to microcrystalline cellulose is about 1:0.30 by weight.

EP-A-1 938 842 relates to a method for preparation of a pharmaceutical composition having improved disintegradability. In the examples active ingredients containing granules comprising mannitol are prepared and mixed among others with microcrystalline cellulose. In any case, the disintegration time is in the range of several minutes.

WO 2007/074472 discloses a mouth dissolving pharmaceutical composition comprising one or more fillers having a certain particle size and 5 to 30 % by weight silicon dioxide. In example 2 an orally disintegrating/dissolving tablet is prepared which comprises mannitol and microcrystalline cellulose in a ratio of about 1:0.12 by weight.

WO 2006/074951 discloses an orally disintegrating pharmaceutical composition which comprises olanzapine or donepezil, mannitol and calcium silicate. The tablets of the examples additionally comprise microcrystalline cellulose whereby the ratio of mannitol to microcrystalline cellulose is below about 1:0.14.

WO 2003/103629 relates to orally administered tablets that disintegrate quickly in the oral cavity in less than 30 seconds, comprising at least 59.5 % mannitol, 10 to 18 % microcrystalline cellulose and the superdisintegrant sodium croscarmellose. Thus, the ratio of mannitol to microcrystalline cellulose is below about 1:0.30.

Most of the prior art oral dispersible tablets require the presence of at least one disintegrant or a specific production processes, such as the co-processing of certain excipients in WO 2007/113856. The requirement of disintegrants in the oral dispersible tablets has the disadvantage of additional costs and possible incompatibilities between the disintegrant and other ingredients. The requirement of certain processing steps results in an increase in preparation costs. Therefore, there is still a need for further oral dispersible tablet formulations which overcome these and other problems of the prior art.

It has now surprisingly been found that tablets with a very short disintegration time in the mouth, a low friability, and a good hardness are obtained when two specific fillers are used in a certain ratio. One of the fillers is selected from sugars and sugar alcohols and the other filler is microcrystalline cellulose. The two fillers are present in a narrow range of 1:0.60 to 1:3.00 by weight. This formulation does not require the addition of a disintegrant. Moreover, an oral dispersible tablet containing these fillers can be prepared without the requirement of any specific processing steps. Advantageously, the oral dispersible tablet of the present invention can be prepared by direct compression.

Thus, the present invention relates to an oral dispersible tablet comprising at least one filler selected from sugars and sugar alcohols, and microcrystalline cellulose, **characterized in that** the ratio of the at least one filler selected from sugars and sugar alcohols to the microcrystalline cellulose is in the range of 1:0.60 to 1:3.00 by weight.

The oral dispersible tablet of the present invention are manufactured as biplane or biconvexe tablets with sharp edges or facet. The ratio between height of the tablet and diameter of the tablets ranges from 1:1.6 to 1:3.2, for example tablets will have a height of 3.8 to 4.8 mm and a diameter of 8 mm.

All values for the hardness and the disintegration time are mean values. The normal range for the hardness of the oral dispersible tablets is mean ± 10 N. The disintegration time has a range ± 5 sec. if not otherwise mentioned.

The oral dispersible tablet according to the present invention exhibits a disintegration time below 30 seconds when measured according to Ph.Eur. 2.9.1 ("Disintegration of tablets and capsules"), Test A with discs.

The oral dispersible tablet of the present invention comprises at least one filler selected from sugars and sugar alcohols. Examples of suitable sugars and sugar alcohols are dextrates, dextrin, dextrose, lactose, maltodextrin, mannitol, isomalt, sorbitol, sucrose, sugars spheres, xylitol, fructose, lactitol, erythritol, maltitol, xylose, glucose, mannose, galactose, maltose, cellobiose, trehalose and raffinose; mannitol, sorbitol, isomalt, xylitol and maltitol being preferred. Most preferably, the oral dispersible tablet of the present invention contains mannitol or isomalt as filler.

If the filler is mannitol, a spray-dried mannitol can be used. The mannitol particles can have a mean diameter between 75 and 200 µm, preferably between 75 and 150 µm and most preferable between 75 and 110 µm.

The second ingredient of the oral dispersible tablet of the present invention is microcrystalline cellulose. Any grade of microcrystalline cellulose commonly used in pharmaceutical compositions an be employed.

The filler selected from sugars and sugar alcohols and the microcrystalline cellulose must be present in a certain ratio in the narrow range of 1:0.60 to 1:3.00 by weight. If the ratio is above 1:0.60 by weight, i.e. if the amount of the sugar or sugar alcohol is increased compared to the amount of the microcrystalline cellulose, the disintegration time of the obtained tablets sharply increases to above 30 seconds making the tablets unsuitable as oral dispersible tablets. If the ration is lower than 1:3.00 by weight, i.e. if the amount of microcrystalline cellulose is increased compared to the amount of sugar or sugar alcohol, hardness and friability of the obtained tablets can be unsatisfactory. Moreover, a high amount of microcrystalline cellulose can result in an unpleasant mouth feeling.

Preferably, the ratio of the at least one filler selected from sugars and sugar alcohols to the microcrystalline cellulose is in the range of 1:0.60 to 1:2.00 by weight, more preferably in the range of 1:0.70 to 1:1.30 by weight. Suitable weight ratios are, for example, 1:0.60, 1:0.70, 1:0.75, 1:1.00, 1:1.15, 1:1.25, 1:1.50, 1:1.75, 1:2.00, 1:2.25, 1:2.50, 1:2.75 and 1:3.00. Each of the exemplified ratios may be combined with any other ratio to form a possible range, such as 1:0.75 to 1:2.00 by weight or 1:0.75 to 1:1.25 by weight.

In addition to the above described at least one filler selected from sugars and sugar alcohols and the microcrystalline cellulose, the oral dispersible tablet of the present invention may further comprise at least one additional filler. There are no particular limitations with regard to this additional filler. The following fillers can be exemplified: calcium carbonate, calcium sulphate, calcium silicate, chitin, chitosan, dibasic calcium phosphate dihydrate, glyceryl palmitostearate, hydrogenated vegetable oil, kaolin, magnesium aluminium silicate, magnesium carbonate, magnesium oxide, polymethacrylates, potassium chloride, powdered cellulose, pregelatinized starch, sodium chloride, starch, talc, and tribasic calcium phosphate. For example, calcium silicate can be used as additional filler in particular in combination with mannitol.

One of the advantages of the oral dispersible tablet of the present invention is that no additional disintegrant is required. In this regard, it is noted that microcrystalline cellulose can function as filler as well as disintegrant. Therefore, in a preferred embodiment the oral dispersible tablet of the present invention does not contain any disintegrant other than microcrystalline cellulose. If one of the above exemplified additional fillers also functions as disintegrant, such as chitin, magnesium aluminium silicate, powdered cellulose, pregelatinized starch or starch, this additional filler is preferably not present in the oral dispersible tablet.

In addition to the above described ingredients, the oral dispersible tablet of the present invention may contain if desired or necessary further common excipients or other ingredients such as taste masking agents, sweeteners, flavours and/or lubricants. Any of these additional ingredients may be present in any suitable amount known to a person skilled in the art.

Common taste masking agents are, for example, cyclodextrines, sodium citrate, sodium hydrogen phosphate, polacriline potassium and the like.

Common sweetening agents are, for example, acesulfame potassium, aspartame, compressible sugar, dextrose, glycerine, lactose, liquid glucose, maltitol solution, mannitol, saccharine, saccharine sodium, sodium cyclamate, sorbitol, sucrose, xylitol and the like. If any sugar or sugar alcohol is used as sweetening agent, it counts to the total amount of sugars and sugar alcohols used as filler in combination with the microcrystalline cellulose.

Common flavours are, for example, bergamot, caramel, cherry, grapefruit, lemon, menthol, orange, peach, peppermint, vanillin and the like.

Common lubricants are, for example, adipic acid, aluminium stearate, calcium stearate, cethyl alcohol, fumaric acid, glyceryl monostearate, glyceryl palmitostearate, glyceryl tripalmitate, hydrogenated castor oil, hydrogenated vegetable oil, light mineral oil, magnesium lauryl sulphate, magnesium stearate, mineral oil, myristic acid, myristic alcohol, palmitic acid, polyethylene glycol, sodium benzoate, sodium lauryl sulphate, sodium stearyl fumarate, stearic acid, stearyl alcohol, talc, triglycerides, zinc stearate and the like.

In one embodiment the oral dispersible tablet according to the invention further comprises an active pharmaceutically ingredient (API) or a nutraceutical ingredient.

Further suitable pharmaceutically active ingredients are:
- Antidiabetics:: Glitazone: Rosiglitazone, Rosiglitazone maleate, Rosiglitazone cholinate, Pioglitazone DPPIV-Inhibitoren: Sitagliptin, Vildagliptin, Alogliptin, Saxagliptin, Denagliptin;
- Antidementia:: Donepezil, Donepezil hydrochloride, Memantine, Memantine hydrochloride, Rivastigmine, Rivastigmine tartrate, Tacrin, Galanthamin;
- Antidepressants:: Amitryptilin, Atomoxetin, Citalopram, Escitalopram, Doxepin, Desipramin, Fluoxetin, Imipramin, Maprotilin, Mianserin, Moclobemid, Mirtazapin, Opipramol, Venlafaxin;
- Antiemetics:: Ondansetron (Ondansetron hydrochloride), Granisetron, Tropisetron, Diphenhydramin, Chlorphenoxamin, Perphenazin, Promethazin, Chlorpromazin, Metoclopramid, Domperidon, Bromoprid;
- Anticonvulsants:: Phenobarbital, Primidon, Phenytoin, Ethosuximid, Mesuximid, Clonazepam, Diazepam, Carbamazepin, Felbamat, Flumazenil, Lamotrigin, Levetiracetam, Oxcarbazepin, Topiramat, Valproinsäure, Zonisamid;
- Antipsychotics:: Aripiprazol, Olanzapin;
- Antimigraine: Medications: Sumatriptan, Rizatriptan, Zolmitriptan, Eletriptan, Frovatriptan, Naratriptan;
- Antiparkinson: Drugs: Rasagilin, Levodopa, Benserazid, Carbidopa, Levodopa/Benserazid (Kombination), Levodopa/Carbidopa (Kombination), Bromocriptin, Cabergolin, Pramipexol, Ropinirol, Selegilin, Entacapon, Biperiden;
- Agents for cardiovascular diseases:: Sartane: Valsartan, Losartan, Telmisartan, Eprosartan, Olmesartan, Candesartan, Irbesartan;
ACE-Inhibitors: Captopril, Enalapril, Lisinopril, Ramipril, Fosinopril, Perindopril, Quinapril, Spirapril;
Ca-Antagonisten: Isradipin, Lacidipin, Lercanidipin, Manidipin, Nimodipin, Trimatazidin;
- Diuretics:: Bendroflumethiazid, Furosemid, Hydrochlorothiazid, Indapamid, Torasemid, Xipamid, Piretanid;
- Agents for incontinence:: Solifenacin, Darifenacin, Tolterodin und Fesoterodin;
- Agents for benign prostatic hyperplasia (BPH):: Tamsulosin, Silodosin;
- Neuroleptics:: Chlorpromazin, Acepromazin, Trifluperazin, Fluphenazin, Chlorprothixen, Flupenthixol, Droperidol, Haloperidol, Fuspirilen, Reserpin, Risperidon, Clozapin, Oxypertin, Sulpirid, Melperon;
- Phosphodiesterase-Inhibitors:: Sildenafil, Tadalafil, Vardenafil;
- Thyroid Therapeutics:: Levothyroxin, Liothyronin;
- Analgesics:: Ibuprofen, Paracetamol, ASS, Diclofenac, Meloxicam, Piroxicam, Naproxen, Flurbiprofen, Fenoprofen, Flufenaminsäure, Mefenaminsäure, Meclofenaminsäure, Indometacin, Felbinac, Tiaprofensäure, Phenazon, Olsalazin;
- Tranquilizer:: Alprazolam, Bromazepam, Citalopram, Escitalopram, Diazepam, Indiplon, Oxazepam, Tetrazepam;
- Others:: Ambrisentan (pulmonal arterial hypertension), Anastrozol, Cinacalcet (hyperparathyroidism), Dapoxetin, (selective serotonin reuptake inhibitor), Duloxetin (serotonin and noradrenalin reuptake inhibitor), Letrozol, Fingolimod (multiple sclerosis), Coffein, Montelukast (antiasthmatics)
and pharmaceutically acceptable salts thereof.

There are no particular limitations on the pharmaceutically active ingredient and the nutraceutical ingredient used in the present invention. Specific examples of the pharmaceutically active ingredient or the nutraceutical ingredient used in the present invention include anti-dementia agents such as donepezil, in particular donepezil hydrochloride, galantamin hydrobromide, rivastigmine tartrate, memantine, in particular memantine hydrochloride, and tacrine; drugs for treating diabetes; anti-anxiety drugs; and vitamins such as ascorbic acid.

There are no particular limitations on the amount in which the pharmaceutically active ingredient or the nutraceutical ingredient is included in the oral dispersible tablet according to the invention. The amount depends on the active ingredient and can be chosen by a person skilled in the art based on his general knowledge.

The oral dispersible tablets of the present invention can, for example, contain the following ingredients and exhibit the following features:

| | | Example A | Example B | Example C |
|---|---|---|---|---|
| Ingredient | Mass per tablet [mg] | Mass per tablet [mg] | Mass per tablet [mg] | Mass per tablet [mg] |
| Sugar or sugar alcohol | 60-240 | 60 | 120 | 200 |
| Microcrystalline cellulose | 60-260 | 120 | 180 | 220 |
| Additional filler | 0-80 | 0 | 20 | 60 |
| Active ingredient | 0-50 | 0.1 | 20 | 50 |
| Taste masking agent | 0-50 | 1 | 20 | 50 |
| Sweetener | 0-1 | 0.25 | 0.5 | 1 |
| Flavour | 0-3 | 0.5 | 1.5 | 3 |
| Lubricant | 0-10 | 2 | 6 | 10 |
| | | | | |
| Friability | < 1% | < 1% | < 1% | < 1% |
| Hardness | 30-70N | 30-70N | 30-70N | 30-70N |
| Disintegration Time | < 30 sec. | < 30 sec. | < 30 sec. | < 30 sec. |

The oral dispersible tablets of the present invention can be manufactured by methods known in the art. For example, by the following steps: sieving the ingredients, mixing except lubricants, adding the lubricant, tabletting on a rotary type or other press, dedusting the tablets, and packaging. Preferably, the tablets are prepared by direct compression.

If the pharmaceutically active ingredient or the nutraceutical ingredient has an unpleasant taste, a taste masking agent and/or flavours can be added. Alternatively, the pharmaceutically active ingredient may be coated by methods known to a person skilled in the art.

The following examples are intended to demonstrate the advantages of the present invention without being limiting. In the examples the hardness of the tablets is measured according to Ph.Eur. 2.9.8 and the friability of the tablets is measured according to Ph.Eur. 2.9.7.

### Reference Examples 1 and 2

The formulations of the tablets according the Ref. Examples 1 and 2 are summarized in table 1. Ref. Example 2 is a typical oral dispersible tablet containing mannitol as filler and crospovidone as disintegrant. The hardness of the tablet is good and the disintegration time is below 30 seconds. If the disintegrant is omitted as in the tablets of Ref. Example 1, the hardness of the tablets is still good but the disintegration time increases to an extent that the tablets are no longer useful as oral dispersible tablets.

**Table 1**

| | | **Ref. Example 1** without disintegrant | **Ref. Example 2** with disintegrant |
|---|---|---|---|
| **Ingredient** | **Function** | **Mass per tablet [mg]** | |
| Calcium silicate | Filler | 35.00 | 35.00 |
| Mannitol | Filler | 134.54 | 134.54 |
| Crospovidone | Disintegrant | - | 16.00 |
| Sodium monohydrogen phosphate | Taste masking agent | 10.00 | 10.00 |
| Saccharin-Na | Sweetener | 0.25 | 0.25 |
| Aroma Peppermint | Flavour | 0.75 | 0.75 |
| Aroma Menthol | Flavour | 0.25 | 0.25 |
| Magnesium stearate | Lubricant | 2.00 | 2.00 |
| | | | |
| Hardness | | 40 N | 40 N |
| Disintegration time | | 100 sec. | 26 sec. |

The manufacture of the tablets of Ref. Examples 1 and 2 follows a standard direct compression method. Alternatively, granulated material may be used. Both wet granulation, either with water as the liquid or with water - alcohol or isopropanol as liquid can be used.

Further, dry granulation methods may be used as well (compaction in a compactor like Gerteis Polygran).

All excipients except magnesium stearate are sieved by use of a 1.0 mm sieve. Magnesium stearate is sieved by use of a 0.5 mm sieve. Calcium silicate is blended with mannitol by use of an drum mixer for 5 min. The remaining excipients except magnesium stearate are filled into the drum. The blend is mixed for 10 min. magnesium stearate is added and then the mixture is blended for 2 min. The mixture is filled into an eccentric tabletting machine and compressed into tablets having a mean hardness of 40 N. The final tablets have a white aspect, the radius of curvature is zero (biplane tablets).

The long mean disintegration time of about 100 seconds for the formulation of Ref. Example 1 without a disintegrant and only calcium silicate and mannitol as fillers necessitates the addition of the disintegrant in Ref. Example 2. The resulting mean disintegration time was 26 seconds.

### Examples 1-5

According to the invention oral dispersible tablets having a very low disintegration time can be obtained by adding microcrystalline cellulose in a distinct ratio to the first filler instead of a disintegrant. This surprising effect is demonstrated by the oral dispersible tablets of Examples 1-5, the formulations of which are summarized in the following table 2.

**Table 2**

| | **Example 1** | **Example 2** | **Example 3** | **Example 4** | **Example 5** |
|---|---|---|---|---|---|
| | ratio Mannitol : Microcrystalline cellulose | | | | |
| | 1:0.74 | 1:0.74 | 1:1.14 | 1:1.14 | 1:1.15 |
| **Ingredient** | **Mass per tablet [mg]** | | | | |
| API | 5.21 | 5.21 | 5.21 | 5.21 | 10.00 |
| Calcium silicate | 35.00 | - | - | - | - |
| Mannitol | 87.18 | 107.32 | 87.18 | 87.18 | 84.00 |
| Microcrystalline cellulose | 64.36 | 79.22 | 99.36 | 99.36 | 97.00 |
| Sodium monohydrogen phosphate | 10.00 | 10.00 | 10.00 | 10.00 | 9.75 |
| Saccharin-Na | 0.25 | 0.25 | 0.25 | 0.25 | 0.25 |
| Aroma Peppermint | 0.75 | 0.75 | 0.75 | 0.75 | 0.75 |
| Aroma Menthol | 0.25 | 0.25 | 0.25 | 0.25 | 0.25 |
| Sodium stearyl fumarate | - | - | 2.00 | 2.00 | 3.00 |
| Magnesium stearate | 2.00 | 2.00 | - | - | - |
| | | | | | |
| Tabletting machine | EK0 | EK0 | EK0 | Korsch XL | Fette 102i |
| Tablet shape | biplane | biplane | biplane | biconvex | biconvex |
| Hardness | 30 N | 37 N | 40 N | 39 N | 40 N |
| Disintegration time | 8 sec. | 15 sec. | 8 sec. | 9 sec. | 9 sec. |
| Friability | 2.5% | 1.1% | 0.4% | n.d. | n.d. |

The manufacture of the tablets of Examples 1-5 follows a standard direct compression method. Alternatively, also granulated material may be used. Both wet granulation, either with water as the liquid or with water - alcohol or isopropanol as liquid can be used. Further, dry granulation methods may be used as well (compaction in a compactor like Gerteis Polygran).

Method of manufacture of the tablets of Examples 1 to 3:

All excipients except sodium stearyl fumarate or magnesium stearate are sieved by use of a 1.0 mm sieve. Sodium stearyl fumarate or magnesium stearate are sieved by use of a 0.5 mm sieve. The API (active pharmaceutical ingredient) is blended with calcium silicate and microcrystalline cellulose by use of a drum mixer for 10 min. The remaining excipients except sodium stearyl fumarate or magnesium stearate are filled into the drum. The blend is mixed for 10 min. Sodium stearyl fumarate or magnesium stearate is added and then the mixture is blended for 2 min. The mixture is filled into an eccentric tabletting machine and compressed into tablets having a mean hardness between 30 and 40 N. The final tablets have a white aspect, the radius of curvature is zero (biplane tablets). The height of the tablets ranged from 3.5 to 4.1 mm. For these tablets the mean disintegration time is less than 20 sec.

Method for manufacture of the tablets of Example 4:

All excipients except sodium stearyl fumarate are sieved by use of a 1.0 mm sieve. Sodium stearyl fumarate is sieved by use of a 0.5 mm sieve. The API is blended with Calcium silicate and microcrystalline cellulose by use of a drum mixer for 10 min. The remaining excipients except sodium stearyl fumarate or Magnesium stearate are filled into the drum. The blend is mixed for 10 min. Sodium stearyl fumarate is added and then the mixture is blended for 2 min. The mixture is filled into a rotary type tablet press and compressed with 2.4 to 2.5 kN main pressure at a speed of 6 000 tablets per hour into tablets having a mean hardness of 39 N. The final tablets have a white aspect, the radius of curvature is 10 mm (biconvex tablets). The height of the tablets ranged from 4.39 to 4.49 mm For these tablets the mean disintegration time is less than 10 sec.

Method of manufacture of the tablets of Example 5:

All excipients except sodium stearyl fumarate are sieved by use of a 1.0 mm sieve. Sodium stearyl fumarate is sieved by use of a 0.5 mm sieve. Mannitol is divided into three parts. The API is blended with the first part of Mannitol by use of a drum mixer for 5 min. Then the second part of Mannitol is added and the mixture is blended for 5 min. After adding the third part and mixing it with the mixture of the previous steps for 5 min this blend is carried over to a high shear granulator. The remaining excipients except sodium stearyl fumarate are filled into the granulator. The blend is mixed for 10 min. Sodium stearyl fumarate is added and then the mixture is blended for 0.5 min. The mixture is filled into a rotary type tablet press and compressed with 6.7 to 6.9 kN main pressure, at a speed of 50 000 tablets per hour, and 0.6-0.7 kN as the prepressure force into tablets, having a mean hardness of 40 N. The final tablets have a white aspect, the radius of curvature is 10 mm (biconvex tablets). The height of the tablets ranged from 4.15 to 4.21 mm. For these tablets the mean disintegration time is less than 10 sec.

### Examples 6-9

**Table 3**

| | **Example 6** | **Example 7** | **Example 8** | **Example 9** |
|---|---|---|---|---|
| | ratio Mannitol : Microcrystalline cellulose | | | |
| | 1:1.17 | 1:1.17 | 1:1.15 | 1:1.15 |
| **Ingredient** | **Mass per tablet [mg]** | | | |
| Donepezil-HCl | 5.00 | 10.00 | - | - |
| Memantine-HCl | - | - | 10.00 | 20.00 |
| Mannitol | 86.20 | 83.183 | 84.00 | 168.00 |
| Microcrystalline cellulose | 100.55 | 97.031 | 97.00 | 194.00 |
| Sodium monohydrogen phosphate | 10.00 | 9.65 | 9.75 | 19.50 |
| Saccharin-Na | 0.25 | 0.241 | 0.25 | 0.50 |
| Aroma Peppermint | 0.75 | 0.724 | 0.75 | 1.50 |
| Aroma Menthol | 0.25 | 0.241 | 0.25 | 0.50 |
| Sodium stearyl fumarate | 2.00 | 1.93 | 5.00 | 10.00 |
| | | | | |
| Tabletting machine | Fette 1200 | Fette 1200 | Fette 1200 | Fette 102i |
| Tablet shape | biconvex | biconvex | biconvex | biconvex |
| Hardness | 41 N | 46 N | 45 N | 40 N |
| Disintegration time | 9 sec. | 5 sec. | 11 sec. | 6 sec. |
| Friability | 0.16 % | 0.33% | n.d. | 0.5% |

Method of manufacture of the tablets of Example 6-9:

All excipients except sodium stearyl fumarate are sieved by use of a 1.0 mm sieve. Sodium stearyl fumarate is sieved by use of a 0.5 mm sieve. Mannitol is divided into three parts. The API is blended with the first part of Mannitol by use of a drum mixer for 5 min. Then the second part of Mannitol is added and the mixture is blended for 5 min. After adding the third part and mixing it with the mixture of the previous steps for 5 min this blend is carried over to a high shear granulator. The remaining excipients except sodium stearyl fumarate are filled into the granulator. The blend is mixed for 10 min. Sodium stearyl fumarate is added and then the mixture is blended for 0.5 min. The mixture of example 6 and 7 is filled into a rotary type tablet press and compressed with 3.8 to 4.3 kN main pressure, at a speed of 80 000 to 120 000 tablets per hour, and 1-1.1 kN as the prepressure force into tablets, having a mean hardness of 40 N. The mixture of example 8 is filled into a rotary type tablet press and compressed with 6.2 kN main pressure, at a speed of 80 000 to 120 000 tablets per hour, and 1.4 kN as the prepressure force into tablets, having a mean hardness of 40 N. The mixture of example 9 is filled into a rotary type tablet press and compressed with 13 kN main pressure, at a speed of 20 000 tablets per hour, and 2.7 kN as the prepressure force into tablets, having a mean hardness of 40 N.

The final tablets of example 6 and 8 have a white aspect, the diameter of the tablets is 8 mm and the radius of curvature is 10 mm (biconvex tablets). The final tablets of example 7 have a pale yellow aspect, the diameter of the tablets is 8 mm and the radius of curvature is 10 mm (biconvex tablets). The final tablets of example 9 have a white aspect, the diameter of the tablets is 12 mm and the radius of curvature is 18 mm (biconvex tablets).

The height of the tablets of example 6 ranged from 4.63 to 4.70 mm. The range of the height was from 4.59 to 4.67 mm for the tablets of example 7. The height of the tablets ranged from 4.31 to 4.41 mm for the tablets of example 8 and from 4.11 to 4.17 mm for the tablets of example 9. For these tablets (example 6-9) the mean disintegration time is less than 10 sec.

### Examples 10 and 11; Reference Example 3

In Examples 10 and 11 the filler mannitol is replaced by isomalt. Moreover, the ratio between the first filler (isomalt) and microcrystalline cellulose is varied from 1:1.17 to 1:2.00.

Reference Example 3 demonstrates that at a ratio between isomalt and microcrystalline cellulose of 1:0.50, i.e. outside the claimed range of 1:0.60 to 1:3.00, tablets having a mean disintegration time of 50 sec. are obtained. These tablets are not suitable as oral dispersible tablets.

**Table 4**

| | **Example 10** | **Example 11** | **Ref. Example 3** |
|---|---|---|---|
| | ratio between Isomalt : Microcrystalline cellulose | | |
| | 1:1.17 | 1:2.00 | 1:0.50 |
| **Name** | **Mass per tablet [mg]** | | |
| Isomalt | 86.05 | 62.13 | 124.27 |
| Microcrystalline cellulose | 100.35 | 124.27 | 62.13 |
| Sodium monohydrogen phosphate | 10.00 | 10.00 | 10.00 |
| Saccharin-Na | 0.25 | 0.25 | 0.25 |
| Aroma Peppermint | 0.75 | 0.75 | 0.75 |
| Aroma Menthol | 0.25 | 0.25 | 0.25 |
| Sodium stearyl fumarate | 2.00 | 2.00 | 2.00 |
| | | | |
| Tablet shape | biplane | biplane | biplane |
| Hardness | 36 N | 44 N | 37 N |
| Disintegration Time | 20 sec. | 8 sec. | 50 sec. |
| Friability | n.d. | 0.03 % | n.d. |

All excipients except sodium stearyl fumarate are sieved by use of a 1.0 mm sieve. Sodium stearyl fumarte is sieved by use of a 0.5 mm sieve. Microcrystalline cellulose and sodium monohydrogen phosphate are blended by use of a drum mixer for 10 min. The remaining excipients except sodium stearyl fumarate are filled into the drum The blend is mixed for 10 min. Sodium stearyl fumarate is added and then the mixture is blended for 2 min. The mixture is filled into a rotary type tablet press and compressed with 1.2 to 2.2 kN main pressure, at a speed of 6 000 tablets per hour, into tablets having a mean hardness between 36 to 44 N. The final tablets have a white aspect, the radius of curvature is zero (biplane talbets). The height of the tablets ranged from 4.15 to 4.21 mm. The disintegration time depends on the ratio of Isomalt to microcrystalline cellulose. For Example 10 the mean disintegration time is 20 sec., for Example 11 it is 8 sec. and for Ref. Example 3 the mean disintegration time is 50 sec.

### Examples 12-15

To investigate the dependency of the disintegration time of the oral dispersible tablet from the concentration of the disintegrant tablets with different amounts of the superdisintegrant Amberlite^{™} IRP88 were produced. The formulations of the tablets are summarized in Table 5 below. There was an optimum concerning disintegration time by use of 9.9 % of Amberlite^{™} IRP88 (example 10). Nevertheless, all formulations have a mean disintegration time less then 20 sec. Therefore, all formulations are useful as oral dispersible tablets.

Examples 12-15 also demonstrate that the mean disintegration time of tablets comprising a superdisintegrant is comparable to the mean disintegration time of the oral dispersible tablets of the present invention without any additional disintegrant. Thus, the oral dispersible tablet according to the invention has the advantage that no additional disintegrant is required in order to obtain the desired mean disintegration time of below 30 sec.

**Table 5**

| | **Example 12** | **Example 13** | **Example 14** | **Example 15** |
|---|---|---|---|---|
| **Name** | **Mass per tablet [mg]** | | | |
| API | 10 | 10 | 10 | 10 |
| Calcium silicate | 35.00 | 35.00 | 35.00 | 35.00 |
| Mannitol | 77.50 | 77.50 | 77.50 | 67.50 |
| Microcrystalline cellulose | 57.50 | 57.50 | 57.50 | 47.50 |
| Amberlite^{™} IRP88 | 10.00 | 15.00 | 20.00 | 50.00 |
| Magnesium stearate | 3.00 | 3.00 | 3.00 | 3.00 |
| | | | | |
| Tablet shape | biplan | biplan | biplan | biplan |
| Hardness | 40 N | 40 N | 40 N | 40 N |
| Disintegration Time | 10 sec. | 10 sec. | 7 sec. | 15 sec. |

The manufacture of the tablets of Examples 8-11 follows a standard direct compression method. Alternatively, also granulated material may be used. Both wet granulation, either with water as the liquid or with water - alcohol or isopropanol as liquid can be used. Further, dry granulation methods may be used as well (compaction in a compactor like Gerteis Polygran).

All excipients except magnesium stearate are sieved by use of a 1.0 mm sieve. Magnesium stearate is sieved by use of a 0.5 mm sieve. Calcium silicate is blended with mannitol by use of an drum mixer for 5 min. The remaining ingredients except magnesium stearate are filled into the drum. The blend is mixed for 10 min. Magnesium stearate is added and then the mixture is blended for 2 min. The mixture is filled into a eccentric machine and compressed into tablets having a mean hardness of 40 N. The final tablets have a white aspect, the radius of curvature is zero (biplane tablets). For all examples the mean disintegration time is less then 20 sec.

## Claims

1. Oral dispersible tablet comprising at least one filler selected from sugars and sugar alcohols, and microcrystalline cellulose, **characterized in that** the ratio of the at least one filler selected from sugars and sugar alcohols to the microcrystalline cellulose is in the range of 1:0.60 to 1:3.00 by weight.

2. Oral dispersible tablet according to claim 1, wherein the at least one filler is selected form the group consisting of dextrates, dextrin, dextrose, lactose, maltodextrin, mannitol, isomalt, sorbitol, sucrose, sugars spheres, xylitol, fructose, lactitol, erythritol, maltitol, xylose, glucose, mannose, galactose, maltose, cellobiose, trehalose and raffinose.

3. Oral dispersible tablet according to claim 1, wherein the filler is mannitol or isomalt.

4. Oral dispersible tablet according to any of the proceeding claims, further comprising at least one additional filler selected from the group consisting of calcium carbonate, calcium sulphate, calcium silicate, chitin, chitosan, dibasic calcium phosphate dihydrate, glyceryl palmitostearate, hydrogenated vegetable oil, kaolin, magnesium aluminium silicate, magnesium carbonate, magnesium oxide, polymethacrylates, potassium chloride, powdered cellulose, pregelatinized starch, sodium chloride, starch, talc, and tribasic calcium phosphate.

5. Oral dispersible tablet according to claim 4, wherein the additional filler is calcium silicate.

6. Oral dispersible tablet according to any of the proceeding claims, which does not contain any disintegrant other than microcrystalline cellulose.

7. Oral dispersible tablet according to any of the proceeding claims, wherein the ratio of the at least one filler selected from sugars and sugar alcohols to the microcrystalline cellulose is in the range of 1:0.60 to 1:2.00 by weight, preferably in the range of 1:0.70 to 1:1.30 by weight.

8. Oral dispersible tablet according to any of the proceeding claims, further comprising a pharmaceutically active ingredient or a nutraceutical ingredient.

9. Oral dispersible tablet according to claim 8, wherein the pharmaceutically active ingredient is donepezil, memantine or a pharmaceutically acceptable salt thereof.

10. Oral dispersible tablet according to any of the proceeding claims, preparable by direct compression.

11. Use of a pharmaceutical composition comprising a pharmaceutically active ingredient, at least one filler selected from sugars and sugar alcohols, and microcrystalline cellulose, wherein the ratio of the at least one filler selected from sugars and sugar alcohols to the microcrystalline cellulose is in the range of 1:0.60 to 1:3.00 by weight for the manufacture of an oral dispersible tablet.

12. Method of preparing an oral dispersible tablet which comprises the steps of blending a pharmaceutically active ingredient, at least one filler selected from sugars and sugar alcohols, and microcrystalline cellulose, wherein the ratio of the at least one filler selected from sugars and sugar alcohols to the microcrystalline cellulose is in the range of 1:0.60 to 1:3.00 by weight, and processing the obtained blend into tablets, preferably by direct compression.
